# EUROPEAN PATENT APPLICATION

(11) **EP 4 437 937 A1**
(43) Date of publication of application: **02.10.2024**
(21) Application number: 24166109.9
(22) Date of filing: 26.03.2024
(51) Int. Cl.: A61B 3/00, A61B 3/135

(54) **SLIT-LAMP MICROSCOPE**

(30) Priority: 30.03.2023 JP 2023056403
(71) Applicant: TOPCON CORPORATION, Tokyo 174-8580 (JP)
(72) Inventor: YOSHIDA, Yuji, Tokyo, 174-8580 (JP); YAMAMOTO, Satoshi, Tokyo, 174-8580 (JP)
(74) Representative: Louis Pöhlau Lohrentz

(57) **Abstract**

A slit-lamp microscope (10) includes an illumination system (12) that is configured to project a slit light onto a subject eye (E); a slit portion (125) that is configured to generate the slit light having a width by passing a light from a light source (121) through a slit between a pair of slit blades (125a, 125b) in the illumination system (12); and a controller (30, 31) that is configured to control the light source (121); wherein the controller (30, 31) is configured to turn the light source (121) off when the pair of slit blades (125a, 125b) is closed.

## Description

### FIELD OF THE INVENTION

The present invention relates to a slit-lamp microscope.

### BACKGROUND

Conventionally, there has been a slit-lamp microscope that includes an illumination system for irradiating a subject eye with a slit light and an observation system for observing the subject eye. In this slit-lamp microscope, the illumination system is provided with a slit portion that makes a slit light with any width by passing the light from a light source between a pair of slit blades (see JP6904776B2).

This conventional slit-lamp microscope is capable of providing a condition in which a space or slit the pair of slit blades is closed to block the light from the light source, thereby not irradiating the subject eye with the slit light. However, in the slit-lamp microscope, even if the pair of slit blades are in contact with each other, a gap may be formed therebetween unless the processing accuracy in producing both slit blades is extremely high, such that the light passes through the gap and then reaches the subject eye. With this, in the slit-lamp microscope, the gap formed between the two slit blades may be recognized by the examinee or examiner, thereby giving him/her an impression of the slit-lamp microscope having a low quality.

The present invention has been made by considering the above problem. An object of the present invention is to provide a slit-lamp microscope that is capable of preventing the light from escaping or streaming through a gap between the two slit blades when the slit has been closed.

### SUMMARY

To achieve the object, a slit-lamp microscope includes an illumination system that is configured to project a slit light onto a subject eye; a slit portion that is configured to generate the slit light having a width by passing light from a light source through a slit between a pair of slit blades in the illumination system; and a controller that is configured to control the light source, wherein the controller is configured to turn the light source off when the pair of slit blades is closed.

### Advantageous Effects

The slit-lamp microscope according to the present invention can prevent the light from escaping through a gap between the pair of the two slit blades when the slit blades have been closed.

### BRIEF DESCRIPTION OF DRAWINGS

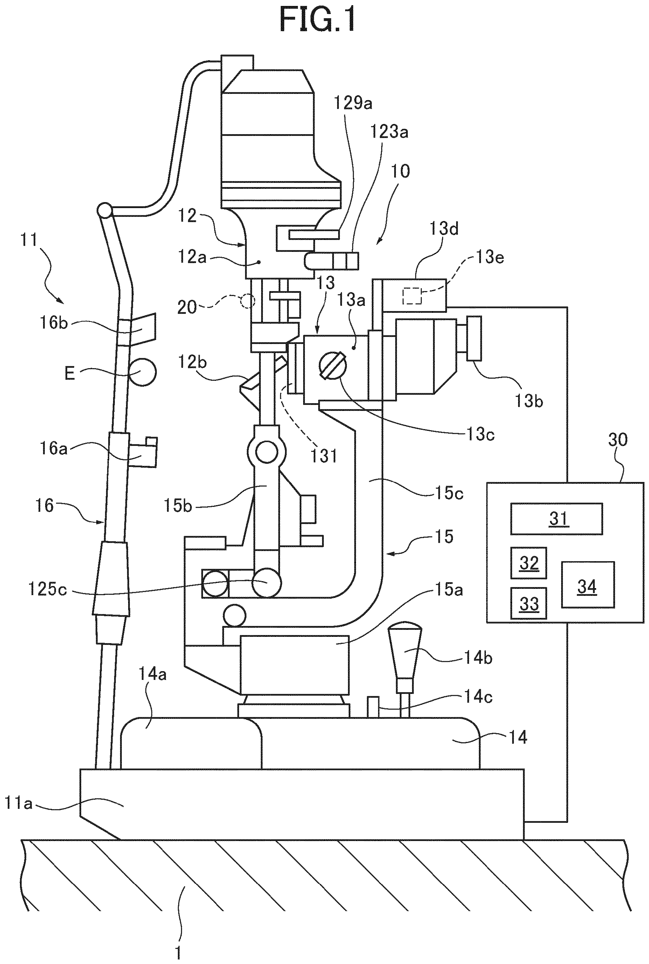

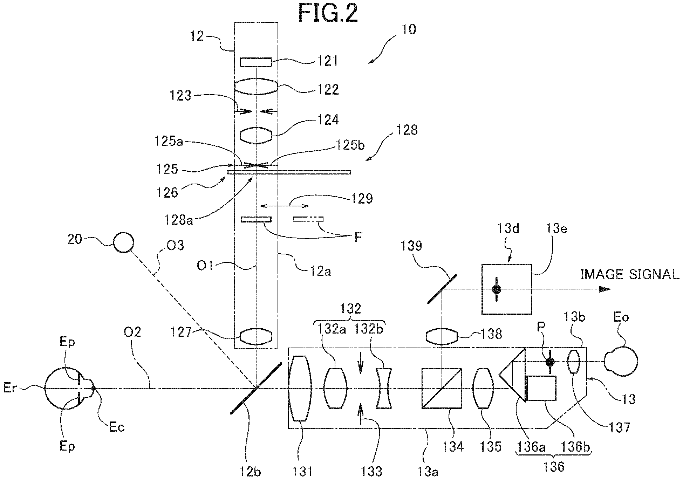

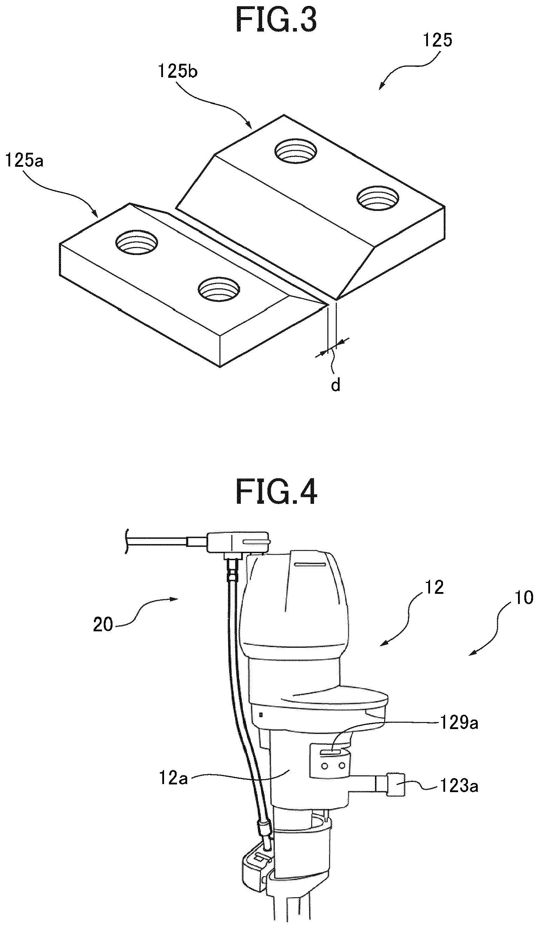

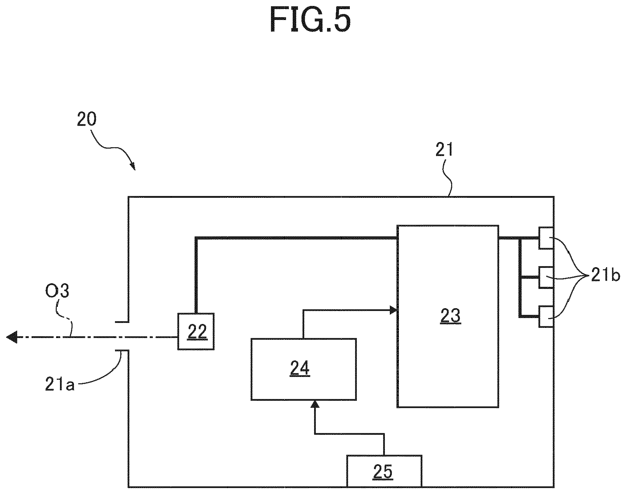

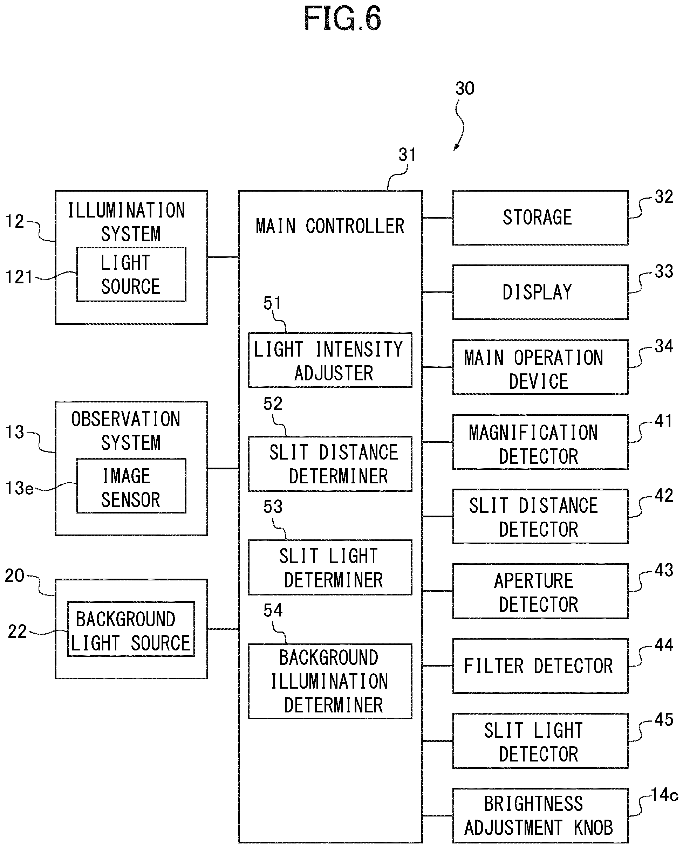

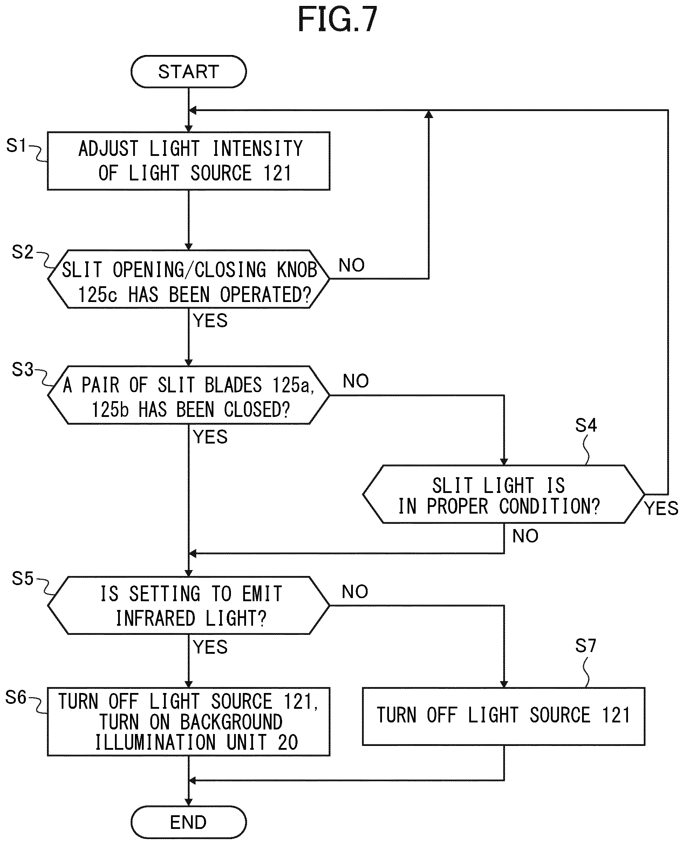

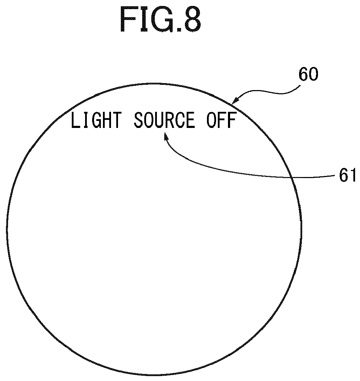

FIG. 1 is a side view illustrating a whole structure of a slit-lamp microscope according to a first embodiment. FIG. 2 is an explanatory view illustrating an optical system of the slit-lamp microscope. FIG. 3 is an explanatory view illustrating a pair of slit blades. FIG. 4 is an explanatory view illustrating a condition that a background illumination unit is attached to a microscope body. FIG. 5 is an explanatory view illustrating a configuration of the background illumination unit. FIG. 6 is a block diagram illustrating a configuration of a controller. FIG. 7 is a flowchart illustrating a flow of a light quantity control in a main controller. FIG. 8 is an explanatory view illustrating a status indicator.

### DESCRIPTION OF EMBODIMENTS

With respect to the use of plural and/or singular terms herein, those having skill in the art can translate from the plural to the singular and/or from the singular to the plural as is appropriate to the context and/or application. The various singular/plural permutations may be expressly set forth herein for the sake of clarity.

A slit-lamp microscope according to an embodiment of the present invention will be described below with reference to FIGS. 1 to 8.

The slit-lamp microscope 10 according to the first embodiment is described with headings "Overall Configuration", "Detailed Configuration of Illumination System", "Detailed Configuration of Observation System", and "Detailed Configuration of Background Illumination Unit", as follows. The slit-lamp microscope 10 is used as an ophthalmologic apparatus that is configured to acquire cross-sectional cornea images by optically cutting out a thin slice of the cornea of the subject eye E with a slit light.

(Overall Configuration) As shown in FIG. 1, the slit-lamp microscope 10 includes a microscope body 11, an illumination system 12, an observation system 13, a background illumination unit 20, and a controller 30. The microscope body 11 is installed on a table 1 and supports the illumination system 12, the observation system 13, and the background illumination unit 20. The microscope body 11 includes a pedestal 11a fixed to the top surface of the table 1, a base 14 provided on the top surface of the pedestal 1 1a, a support 15 provided on the top surface of the base 14, and a chin rest 16 supported on the pedestal 11a. The base 14 is provided relative to the pedestal 11a to be movable in frontward and rearward directions and leftward and rightward directions as viewed from the subject eye E through a movement mechanism 14a. The base 14 is moved by tilting an operation handle 14b. The support 15 on the top surface of the base 14 is moved in vertical directions by rotating the operation handle 14b around its axis.

The support 15 includes a pedestal 15a, a first support arm 15b, and a second support arm 15c. The pedestal 15a is provided on the top surface of the base 14, and the first support arm 15b and the second support arm 15c extend upward from the pedestal 15a. Herein, each of the first support arm 15b and the second support arm 15c are independently rotatable around a coaxial vertical axis.

The first support arm 15b includes an upper part to which an illumination system housing 12a is attached to support the illumination system 12. The illumination system housing 12a accommodates the illumination system 12 therein. The first support arm 15b is manually rotatable, and the rotation of the first support arm 15b rotates the illumination system housing 12a around the subject eye E. With this, it is possible to change the slit-light projection direction relative to the subject eye E. The first support arm 15b may be configured to rotate upward or downward. By having such a configuration, the first support arm 15b can change the elevation angle or depression angle of the slit light to the subject eye E. The second support arm 15c includes an upper part to which an observation system housing 13a is attached to support the observation system 13. The observation system housing 13a accommodates the observation system 13 therein. The second support arm 15c is rotatable, and the rotation of the second support arm 15c rotates the observation system housing 13a around the first support arm 15b. With this, it is possible to change the observation direction of the observation system 13 relative to the subject eye E.

The first and second support arms 15b, 15c may be configured to electrically and automatically be rotatable, respectively. In this case, it is necessary to provide an arm drive mechanism including an actuator that generates a drive force to rotate the first and second support arms 15b, 15c, and a transmission mechanism to transmit the drive force. As the actuator, a motor such as a stepping motor (pulse motor) may be used, for example. As the transmission mechanism, a combination of gearwheels, rack and pinion, or the like may be used, for example.

The chin rest 16 is disposed at a position in front of the observation system housing 13a. The chin rest 16 includes a chin rest portion 16a and a forehead rest portion 16b for stabilizing the face (head) of the examinee. In the slit-lamp microscope 10, the examiner observes the subject eye E in a condition that the examinee facing the table 1 placed his/her face (i.e., chin and forehead) on the chin rest portion 16a and the forehead rest portion 16b.

The illumination system 12 projects the slit light towards the subject eye E. The illumination system 12 is accommodated in the illumination system housing 12a. The intensity of the slit light can be changed by operating a brightness adjustment knob 14c provided on the base 14. The slit light refers to an illumination light for observing the cornea or fundus of the subject eye E, and the illumination light has an illumination region which has been formed into a band shape by blocking a part of the illumination region. Below the illumination system housing 12a, a mirror 12b is disposed which reflects the slit light projected from the illumination system 12 towards the subject eye E. The mirror 12b is attached to the first support arm 15b. The mirror 12b also makes it possible to reflect the background light projected from the background illumination unit 20 toward the subject eye E.

The observation system 13 observes and photographs or images the reflection light reflected by the subject eye E. Herein, the reflection light includes not only the slit light and the background light reflected by the subject eye E but also various lights such as scattered light from the subject eye E and its surroundings. In the first embodiment, these various lights are correctively referred to as "the reflection light". The observation system 13 is accommodated in the observation system housing 13a. At a terminal end of the observation system housing 13a, an eyepiece portion 13b is provided. The examiner observes the subject eye E with the naked eyes by looking through the eyepiece portion 13b. On a side surface of the observation system housing 13a, an observation magnification operation knob 13c is disposed for changing the observation magnification.

An imaging device 13d for photographing or imaging the subject eye E is detachably connected to the observation system housing 13a. The imaging device 13d includes an image sensor 13e. The image sensor 13e is a photoelectric conversion element to detect light and output electric signals (image signals). The image sensor 13e outputs image signals to the controller 30. For the image sensor 13e, for example, a CCD (Charge Coupled Device) image sensor or a CMOS (Complementary Metal Oxide Semiconductor) image sensor is used.

The background illumination unit 20 illuminates by the background light a surrounding region of a projection region of the slit light from the illumination system 12 to the subject eye E. It is sufficient that the projection region by the background illumination unit 20 includes at least this surrounding region and it may partly overlap with the projection region by the illumination system 12.

The controller 30 is connected to the slit-lamp microscope 10 (microscope body 11). The controller 30 controls each part of the slit-lamp microscope 10 and conducts various control processes and calculations. The controller 30 is built in the base 14 in the first embodiment but may be accommodated in the microscope body 11 or the like. Furthermore, the controller 30 may be arranged to be separate from the microscope body 11 and is not limited to the configuration of the first embodiment.

(Detailed Configuration of Illumination System) As shown in FIG. 2, the illumination system 12 includes, on an illumination optical axis O1, a light source 121, a relay lens 122, an illumination diaphragm 123, a condensing lens 124, a slit portion 125, a field diaphragm 126, and an imaging lens 127. The light source 121 is a light source of the slit light for observing the cornea and fundus of the subject eye E. The light source 121 is configured to output at least visible light. The first embodiment uses both a light source (halogen lamp, LED (Light Emitting Diode), or the like) that outputs stationary or continuous light, and a light source (xenon lamp, LED, or the like) that outputs flashlight. The illumination system 12 may be configured by a single light source or a plurality of light sources. In the illumination system 12, a plurality of monochromatic light sources may be provided separately, such as in the case where a light source for observing the cornea and a light source for observing the fundus are provided separately. The light source 121 is capable of changing the luminous intensity (amount of light) in accordance with the magnitude of the applied power under the control of the main controller 31, which is described below.

The illumination diaphragm 123 blocks a peripheral part of the illumination light and transmits only a central part of the illumination light. The illumination diaphragm 123 is capable of changing the size (diameter and aperture area) of its light-transmitting part, thereby adjusting the illuminance of an illuminated part. The illumination diaphragm 123 is capable of reducing the reflection of the illumination light by cornea Ec and/or crystalline lens of the subject eye E and adjusting the brightness of the illumination light. The illumination diaphragm 123 according to the first embodiment includes a diaphragm knob 123a provided on the illumination system housing 12a (see FIG. 1). The diaphragm knob 123a is linked by a link mechanism to a diaphragm frame portion, which forms the light-transmitting part, and is configured to change the size of the light-transmitting part according to a rotational operation on itself. The diaphragm knob 123a may be configured to be freely adjustable by a manual operation with another configuration or to perform an electric adjustment under the control of the main controller 31.

The slit portion 125 is used for generating the slit light. The slit portion 125 includes a pair of slit blades 125a, 125b that are opposed to each other with a predetermined distance therebetween (see FIGS. 2 and 3). The slit portion 125 is capable of moving the pair of slit blades 125a, 125b to adjust the distance d (slit width) between the slit blades 125a, 125b to any size (slit width). The slit portion 125 according to the first embodiment includes a slit opening/closing knob 125c provided at a lower part of the first support arm 15b (see FIG. 1).

The slit opening/closing knob 125c is linked to the pair of slit blades 125a, 125b by a link mechanism, and a rotational operation to the slit opening/closing knob 125c is transmitted such that the two slit blades 125a, 125b are moved in an approaching direction or a separating direction relative to each other. Therefore, in the slit portion 125, the two slit blades 125a, 125b approach or separate from each other by the rotational operation to the slit opening/closing knob 125c according to the rotational direction of the slit opening/closing knob 125c to have any distance d therebetween. Therefore, the slit portion 125 can change the width of the slit light in accordance with the rotational operation of the slit opening/closing knob 125c. The slit portion 125 may be configured to freely adjust the distance d between the slit blades 125a, 125b by a manual operation with another configuration or to perform an electric adjustment under the control of the main controller 31.

The slit opening/closing knob 125c according to the first embodiment makes it possible to conduct a rotational operation from a fully opened rotational position at which the distance d becomes maximum to a fully closed rotational position at which the distance d becomes minimum. In the slit opening/closing knob 125c, when the rotation resistance (force resisting the rotation) against the rotational operation is at the fully closed rotational position, this position is different from those at other positions. Therefore, in the slit opening/closing knob 125c, the force required for the rotational operation becomes larger as the rotation resistance increases, and the force required for the rotational operation becomes smaller as the rotation resistance decreases. Thereby, in the slit opening/closing knob 125c, it is possible to recognize the fully closed rotational position by the feeling or sensation of the hand during the rotational operation, that is, the pair of slit blades 125a, 125b has moved to the fully closed position. The change of the rotational resistance against the rotational operation is made possible by an exemplary configuration that a projection in the form of a plate spring is formed on one of the slit opening/closing knob 125c and the first support arm 15b (or its support portion) on which the knob 125c is rotatably supported, and a recess for receiving the projection is formed on the other of them, thereby making the projection fitted into the recess at the fully closed rotational position. In such a configuration, it is possible to obtain a so-called click sensation at the fully closed rotational position. Therefore, the examiner can conduct the rotational operation without an unpleasant feeling and can recognize the fully closed rotational position by only the click sensation. As long as it can recognize the fully closed rotational position by the feeling in the hand or sensation, the change of the rotational resistance against the rotational operation may be achieved by another configuration, and it is not limited to the configuration of the first embodiment.

The illumination system 12 has a luminous flux control portion 128 that is capable of changing the area of an aperture 128a that allows a luminous flux from the light source 121 to pass therethrough. The luminous flux control portion 128 limits the illumination range on the subject eye E by blocking a part of the luminous flux from the light source 121.

The illumination system 12 has a filter portion 129. The filter portion 129 is configured to select any filter F and to insert (dispose) it into or remove it from an optical path. The filter F consists of an optical element that removes or weakens a specific component of the illumination light. The filter F is, for example, a fluorescent filter (a filter (blue filter) that particularly transmits a wavelength component corresponding to blue color) used for improving contrast in a fluorescent observation of the subject eye E. As the filter F, it is possible to use, for example, a red-free filter (green filter) that makes it easy to observe blood vessels, a light-reducing filter (ND filter) that lowers the light quantity without changing color, or the like.

The filter portion 129 according to the first embodiment has a filter switch lever 129a installed on the illumination system housing 12a to insert the selected filter F (a fluorescent filter, red-free filter, light-reducing filter, etc.) into and remove it from the optical path of the illumination system 12. The filter switch lever 129a is linked to each filter F (its support frame) through a link mechanism and inserts any filter F into the optical path in accordance with the position of a slide operation conducted on itself. As long as the filter portion 129 can insert any filter F into and remove it from the optical path, such operation may be manually conducted by another configuration or electrically conducted under the control of the main controller 31, which will be described later.

(Detailed Configuration of Observation System) The observation system 13 includes a pair of left and right optical systems. The examiner can observe the subject eye E with both eyes with the left and right optical systems of the observation system 13. The symbol Ec denotes the cornea of the subject eye E, the symbol Ep denotes the iris, and the symbol Er indicates the fundus. The symbol Eo denotes the examiner's eye. In the observation system 13, the left and right optical systems have generally similar configurations. Therefore, in the description below and FIG. 2, only one of them will be described. As shown in FIG. 2, each optical system of the observation system 13 includes an objective lens 131, a magnification optical system 132, a diaphragm 133, a beam splitter 134, an imaging lens 135, a prism unit 136, and an eyepiece lens 137, which are provided on the observation optical axis O2. The beam splitter 134 may be provided in only one of the left and right optical systems or both of them.

The eyepiece lens 137 is provided in the eyepiece portion 13b. In the observation system 13, an image of an observation part is formed on an imaging position P by the imaging lens 135. The examiner (examiner's eye Eo) observes the image on the imaging position P through the eyepiece lens 137.

The magnification optical system 132 changes the magnification (angle of view) of observation images and photographed images of the subject eye E, which are taken by the eyepiece lens 137. The magnification optical system 132 consists of a plurality of magnifying lens groups that may be selectively inserted with respect to the observation optical axis O2. Each of the magnifying lens groups includes a plurality (two in FIG. 2) of magnifying lenses 132a, 132b, and the magnifying lens groups provide different magnifications, respectively. In the magnification optical system 132, a desired magnification is set by a plurality of lenses of one magnifying lens group, which are arranged on the observation optical axis O2. In this magnification optical system 132, the change of magnification, that is, the replacement of the magnifying lens group to be arranged on the observation optical axis O2 can be conducted by operating the observation magnification operation knob 13c. In the magnification optical system 132, the magnification change (replacement of the magnifying lens group) may be electrically conducted by using a switch, etc. (not shown in the drawings) under the control of the after-mentioned main controller 31.

The beam splitter 134 divides the light traveling along the observation optical axis O2 into two. The light penetrated the beam splitter 134 is guided to the subject eye Eo via the imaging lens 135, the prism unit 136, and the eyepiece lens 137. The prism unit 136 includes two optical elements 136a, 136b to invert the image and change the width between the left and right observation optical axes O2 to correspond to the width of the examiner's eyes. Meanwhile, the light reflected by the beam splitter 134 is guided to the image sensor 13e of the imaging device 13d via an imaging lens 138 and a mirror 139. The image sensor 13e detects this reflected light and generates image signals.

(Detailed Configuration of Background Illumination Unit) The background illumination unit 20 illuminates the background of the observation image in the observation system 13. As shown in FIG. 4, the background illumination unit 20 according to the first embodiment is formed separate from the microscope body 11 and can be attached to any position (e.g., to the illumination system housing 12a in the example shown in FIG. 4) of the microscope body 11. As its attachment means, it is possible to use a magnet, a suction cup, an adhesive sheet, a hook and loop fastener, etc. alone or in combination. It is optional to provide a configuration in which the microscope body 11 (illumination system housing 12a) is provided with a holder to hold the background illumination unit 20 (the after-mentioned unit case 21), thereby attaching the background illumination unit 20 to the microscope body 11.

As shown in FIG. 5, the background illumination unit 20 includes a unit case 21, a background light source 22, a battery 23, a background light controller 24, and a background light operation device 25. By attaching the background illumination unit 20 to the illumination system housing 12a, a background illumination optical axis O3 from the background light source 22 is merged with the illumination optical axis O1 (observation optical axis O2) (see FIGS. 2 and 4). The unit case 21 accommodates therein the background light source 22, the battery 23, the background light controller 24, and the background light operation device 25. The unit case 21 includes an opening 21a that can transmit the background light projected from the background light source 22, and a charging terminal 21b for charging the battery 23. The charging terminal 21b is exposed to the outside of the unit case 21. The unit case 21 can be formed into rectangular, cylindrical, triangular pyramidal, or any other shape.

The background light source 22 is a light source of the background light that illuminates the surroundings of a projection region of the slit light. The background light source 22 is capable of outputting visible light used for naked-eye observation and photography. For example, a halogen lamp, LED, etc. can be used therefor. The background light source 22 is capable of changing its luminous intensity (light quantity) depending on the amount of the applied power. The background light source 22 according to the first embodiment is capable of outputting an infrared light used for observing and photographing the meibomian gland so that it is configured to output the light by suitably selecting the visible light source or the infrared light source. Alternatively, the background light source 22 may be configured to output the light from only one of the visible light source and the infrared light source.

The battery 23 is a source of electric power supply to the background light source 22, the background light controller 24, etc. In the first embodiment, the battery 23 is a secondary battery that can be repeatedly used by charging through the charging terminal 21b. As such battery 23, a nickel metal hydride battery or a lithium-ion battery may be used.

The background light controller 24 includes a CPU and memory and controls the background light source 22 based on commands input from the background light operation device 25 and the after-mentioned main controller 31. The background light controller 24 controls the amount of power applied from the battery 23 to the background light source 22 and suitably changes the ON/OFF state of the background light source 22, luminous intensity (intensity of the background light), and the like. The background light operation device 25 is an operation device operated by the examiner, etc. and includes a switch(es), a button(s), dial(s), etc. The background light operation device 25 is provided on the surface of the unit case 21 to be exposed and is operable by the examiner. The background light operation device 25 can input any command according to the operation to the background light controller 24. For example, it can input a command to change the ON/OFF state, light intensity, etc. of the background light source 22.

The background illumination unit 20 may be configured to provide a lens that converges the background light projected from the background light source 22. The background illumination unit 20 may be configured to illuminate the background without passing through the mirror 12b.

(Configuration of Controller) The controller 30 that controls the slit-lamp microscope 10 will be described with reference to FIGS. 2, 6, etc. The controller 30 includes the main controller 31 that uses a CPU (Central Processing Unit). The main controller 31 acquires necessary information and suitably outputs control commands to the illumination system 12, the observation system 13, and the background illumination unit 20 (its background light controller 24) of the slit-lamp microscope 10. The control program is stored in advance in the after-mentioned storage 32. The control commands from the main controller 31 are realized by the cooperation of the control program and hardware. To the main controller 31, the storage 32, a display 33, a main operation device 34, and the brightness adjustment knob 14c are connected.

The main controller 31 is configured to include a microprocessor, RAM, ROM, a hard disk drive, and the like. In the hard disk drive, the control program is stored in advance. The main controller 31 is operated by the cooperation of the control program and the above-mentioned hardware. The main controller 31 performs overall control of the slit-lamp microscope 10 (illumination system 12, observation system 13, background illumination unit 20, etc.) based on input operations to the main operation device 34 and the brightness adjustment knob 14c. The main controller also reads data stored in the storage 32 and writes data to the storage 32.

The main controller 31 (its light intensity adjuster 51) adjusts the amount of power applied to the light source 121 of the illumination system 12 in accordance with the rotational operation to the brightness adjustment knob 14c, thereby changing the light intensity (light quantity) of the light source 121. Therefore, the main controller 31 functions as a controller that controls the light source 121. The main controller 31 also conducts control of the charge accumulation time, control of the light receiving sensitivity, control of frame rate, and the like in the image sensor 13e of the observation system 13, and acquires image signals generated by the image sensor 13e. Furthermore, the main controller 31 can conduct control of the background light controller 24, that is, control of operation of the background light source 22 of the background illumination unit 20 through the background light controller 24. The main controller 31 receives operation (its signals) conducted on the background light operation device 25 of the background illumination unit 20. The main controller 31 changes the light intensity (light quantity) of the background light source 22 by adjusting the amount of power applied to the background light source 22 in accordance with, for example, the operation conducted on the main operation device 34 and the background light operation device 25.

The storage 32 is configured to include ROM (Read Only Memory), RAM (Random Access Memory), a hard disk drive, and the like, and various data and computer programs can be written into and read out of it.

The display 33 is formed by LCD (Liquid Crystal Display) or the like and displays various information by receiving control commands from the main controller 31. The display 33 is installed on the base 14 or in its vicinity to be visible to the examiner. The display 33 may be installed on the microscope body 11 or in other locations, as long as it is visible to the examiner.

The main operation device 34 is formed of operation devices and input devices, such as the operation panel, switch, button, dial, trackball, mouse, keyboard, etc. The main operation device 34 is operated by the examiner, and any commands in response to the operation are input to the main controller 31. The operation handle 14b installed on the base 14 may also be included in the main operation device 34. The display 33 and the main operation device 34 may be separately provided, but they may be integrated at least in part by using a touch screen, etc.

To the main controller 31, a magnification detector 41, a slit distance (slit width) detector 42, an aperture detector 43, a filter detector 44, and a slit light detector 45 are connected. These five detectors output the detection results (their signals), which are detected by themselves, to the main controller 31.

The magnification detector 41 detects the magnification set in the magnification optical system 132 of the observation system 13, that is, the state of switching of the magnification lens group. The magnification detector 41 can be configured, for example, to provide a readout portion (e.g., a tag) on a support frame or the like for supporting each magnification lens to be moved, and to provide a detector for detecting the readout portion of the magnification lens on the optical path. The magnification detector 41 is not limited to the configuration of the first embodiment but also may be another configuration, as long as it detects magnification in the magnification optical system 132 and outputs detected values to the main controller 31.

The slit distance detector 42 detects the distance (slit width) between the two slit blades 125a, 125b in the slit portion 125. The slit distance detector 42 may be configured, for example, to use a position sensor to detect the positions of the two slit blades 125a, 125b. The slit distance detector 42 may be configured to use a position sensor to detect the positions of driving points of both slit blades 125a, 125b in the slit portion 125. Furthermore, the slit distance detector 42 may also be configured to use a light intensity sensor that detects the light intensity projected from between both slit blades 125a, 125b, and to make a calculation from the relationship between the detection result and the light intensity of the light source 121. In addition, the slit distance detector 42 may also be configured to conduct a determination from the width of the projected slit light based on the image signals (acquired image) from the image sensor 13e. The slit distance detector 42 is not limited to the configuration of the first embodiment but also may be another configuration, as long as it detects the distance (slit width) between the two slit blades 125a, 125b in the slit portion 125 and outputs detected values to the main controller 31. Thus, the slit distance detector 42 functions to detect whether or not the pair of slit blades 125a, 125b is closed.

The aperture detector 43 detects the size (diameter, aperture area) of a light transmitting part in the illumination diaphragm 123 of the illumination system 12. The aperture detector 43 may be configured, for example, to include a position sensor in a mechanism that changes the size of the light-transmitting part of the illumination diaphragm 123. The aperture detector 43 may also be configured to use a light intensity sensor that detects the light intensity of the light transmitted through the light transmitting part and to make a calculation from the relationship between the detection result and the light intensity of the light source 121. The aperture detector 43 is not limited to the configuration of the first embodiment but also may be another configuration, as long as it detects the size (diameter, aperture area) of the light transmitting part in the illumination diaphragm 123 and outputs detected values to the main controller 31.

The filter detector 44 detects whether or not in the illumination system 12 the filter F is inserted into the optical path by the filter portion 129 and also detects the type of the filter F when inserted. The filter detector 44 may be configured, for example, to include a readout portion (e.g., a tag) on a support frame or the like for supporting each filter F, and to include a detector for detecting the readout portion of the filter F on the optical path. The filter detector 44 is not limited to the configuration of the first embodiment but also may be another embodiment, as long as it detects whether or not the filter F is inserted into the optical path and detects the type of the filter F when inserted.

The slit light detector 45 detects the state of the slit light projected toward the subject eye E. This state of the slit light means whether or not the slit light is in a desired single band form with a set length. The slit blades 125a, 125b form the slit light by the light from the light source 121 that passes through the distance d. Accordingly, the light from the light source 121 is blocked by entirely contacting the slit blades 125a, 125b each other, that is the eliminating the distance d between the slit blades 125a, 125b. However, high precision is required to entirely contact the slit blades 125a, 125b each other to prevent the light from leaking from the light source 121. Therefore, it is difficult to make the distance d between the slit blades 125a, 125b completely uniform for preventing the leakage of the light from the light source 121, and accordingly, even when the two slit blades 125a, 125b are brought into contact with each other, the contact of the slit blades 125a, 125b becomes partial contact. However, the variation of the distance d between the two slit blades 125a, 125b is actually extremely small. Therefore, the slit blades 125a, 125b do not cause any problems except when the slit blades 125a, 125b contact each other to block the light. When the slit blades 125a, 125b contact with each other or the distance between the slit blades 125a, 125b becomes close to the contact, the slit light may not be seen as a single straight band but may be seen as an interrupted band or multiple aligned bands.

The slit light detector 45 detects whether the slit light is in an intended single straight band, or in an unintended band or bands such as the interrupted band, or the multiple aligned bands. The slit light detector 45 can be configured, for example, to conduct a determination from the width of the projected slit light, based on the image signals (acquired image) from the image sensor 13e. The slit light detector 45 is not limited to the configuration of the first embodiment but may be another configuration as long as it detects the state of the slit light and outputs the main controller 31.

The main controller 31 may be configured to also change the diaphragm 133 (its stop or aperture value) as a control of the observation system 13 in accordance with operation on the main operation device 34 or the like. Furthermore, the main controller 31 may be configured to separately rotate the first and second support arms 15b, 15c in accordance with the operation on the main operation device 34 by controlling the arm drive mechanism.

(Configuration of Light Intensity Adjustment) The main controller 31 according to the first embodiment includes the light intensity adjuster 51, a slit distance determiner 52, a slit light determiner 53, and a background illumination determiner 54. The light intensity adjuster 51 is one to adjust the magnitude of the power applied to the light source 121. Basically, it adjusts the magnitude of the applied power in accordance with the operation on the brightness adjustment knob 14c or the like. As described hereinafter, the light intensity adjuster 51 stops the electric power application to the light source 121 according to the detection result from the slit distance determiner 52 or the detection result from the slit light determiner 53.

Here, the light intensity adjuster 51 basically adjusts the range of rotational operation of the brightness adjustment knob 14c so that the light intensity ranges from zero upon turning off to the maximum, and changes the applied power so that the light intensity increases or decreases in proportion to the rotational movement of the brightness adjustment knob 14c. As described hereinafter, the light intensity adjuster 51 stops the power application to the light source 121 when the slit distance determiner 52 has determined that the slit blades 125a, 125b are closed, or when the slit light determiner 53 has determined that the slit light is not in a proper condition. As described hereinafter, under the condition that the power application to the light source 121 is stopped, when the slit distance determiner 52 has determined that the slit blades 125a, 125b are opened, the light intensity adjuster 51 starts the power application to the light source 121 and adjusts the magnitude of the applied power in accordance with the operation on the brightness adjustment knob 14c or the like.

Based on the detection result from the slit distance detector 42, the slit distance determiner 52 determines the distance d between the two slit blades 125a, 125b in the slit portion 125. The slit distance determiner 52 according to the first embodiment determines whether or not the acquired or detected distance d is less than or equal to a set distance ds. In the slit portion 125, the set distance ds is set, based on a position where the slit blades 125a, 125b are closed in design. Therefore, the fact that the acquired distance d is equal to the set distance ds means that the two slit blades 125a, 125b are in a positional relationship that they are set as being closed, irrespective of whether or not they are actually closed. The slit distance determiner 52 determines from the detection result obtained by the slit distance detector 42 that the distance d has become equal to the set distance ds as a result of the movement of the two slit blades 125a, 125b relative to each other to the set closed position.

The slit light determiner 53 determines whether or not the slit light is in a proper condition based on the detection result from the slit light detector. This proper condition means that the slit light is substantially in an intended single straight band form. In contrast, an improper condition means that the slit light is an unintended one such as a band form interrupted in the middle or a plurality of separate, aligned, band-like portions.

The background illumination determiner 54 determines whether or not the background illumination unit 20 (background light source 22) is set to emit infrared light based on the signals from the background light controller 24 of the background illumination unit 20. The background illumination determiner 54 according to the first embodiment is configured to be able to select a visible light source and an infrared light source. Therefore, it determines whether or not the background illumination unit 20 is attached to a proper position and is in an operable condition. If determined that it is attached thereto and is in the operable condition, it determines whether or not the infrared light source is selected. In case that the background illumination unit 20 is configured to be able to output only an infrared light source, the background illumination determiner 54 may determine whether or not the background illumination unit 20 is attached thereto and whether or not the background illumination unit 20 is in an operable condition.

(Light Intensity Adjustment Control) Next, the processing structure of the light intensity control conducted in the main controller 31 will be described with reference to the flowchart shown in FIG. 7. The light intensity control processing is started when the power switch of the slit-lamp microscope 10 is turned on and is repeatedly conducted until the power switch is turned off.

In Step S1, the light intensity of the light source 121 is adjusted and then the process proceeds to Step S2. In Step S1, the light intensity adjuster 51 changes the applied power in accordance with the rotational operation on the brightness adjustment knob 14c, that is, the rotational position of the brightness adjustment knob 14c, to adjust the light intensity of the light source 121. In Step S1, the light source 121 is kept turned off even if the rotational operation is conducted on the brightness adjustment knob 14c, in case that the light source 121 is turned off in Steps S6 and S7 after determining that the pair of slit blades 125a, 125b is closed in Step S3 or that the slit light is not in a proper condition in Step S4.

In Step S2, it determines whether or not the slit opening/closing knob 125c was operated. If YES, it proceeds to Step S3. If NO, it returns to Step S1. In Step S2, it determines whether or not the distance (slit width) between the two slit blades 125a, 125b detected by the slit distance detector 42 is changed. If YES in Step S2, in case that the light source 121 is turned off by Step S7, the light source is turned on. Furthermore, if YES in Step S2, in case that the light source 121 is turned off while the background illumination unit 20 is turned on by Step S6, the light source 121 is turned on and the background illumination unit 20 is turned off. Here, in case that the light source 121 is turned off by Steps S6 and S7, there is a situation after determining that the pair of slit blades 125a, 125b is closed by Step S3 or that the slit light is not in a proper condition by Step S4. Therefore, the determination that the slit opening/closing knob 125c was operated means that the pair of slit blades 125a, 125b was opened. Therefore, in Step S2, as the pair of slit blades 125a, 125b was opened, the light source 121 is turned on. At this time, in Step S2, the light source 121 is turned on with a power according to the rotational position of the brightness adjustment knob 14c, that is, with the brightness set by the brightness adjustment knob 14c.

In Step S3, it determines whether or not the pair of slit blades 125a, 125b is closed. If YES, it proceeds to Step S5. If NO, it proceeds to Step S4. In Step S3, the slit distance determiner 52 determines whether or not the distance d between the two slit blades 125a, 125b is less than or equal to the set distance ds.

In Step S4, it determines whether or not the slit light is in a proper condition. If YES, it returns to Step S1. If NO, it proceeds to Step S5. In Step S4, the slit light determiner 53 determines whether or not the slit light is in the proper condition. In a situation where the pair of slit blades 125a, 125b is opened to turn on the light source 121 in Step S2 after the light source 121 is turned off by Steps S6 and S7, it is determined as NO in Steps S3 and S4 and the process returns to Step S 1.

In Step S5, it determines whether or not it is set to emit an infrared light. If YES, it proceeds to Step S6. If NO, it proceeds to Step S7. In Step S5, the background illumination determiner 54 determines whether or not it is set to emit an infrared light in the background illumination unit 20 and further determines in the first embodiment whether or not the infrared light source is selected in the background illumination unit 20.

In Step S6, the light source 121 is turned off and the background illumination unit 20 is turned on, thereby terminating this light intensity control process. In Step S6, the light intensity adjuster 51 stops the power application to the light source 121 and the main controller 31 outputs a signal for turning on the background light source 22 to the background light controller 24.

In Step S7, the light source 121 is turned off to terminate this light intensity control process. In Step S7, the light intensity adjuster 51 stops the power application to the light source 121.

(Configuration of Status Indicator) As shown in FIG. 8, in the observation system 13 of the slit-lamp microscope 10, a status indicator 60 is provided. The status indicator 60 indicates whether the light source 121 is turned on or off, and can be seen by the examiner as an observer. The status indicator 60 according to the first embodiment uses a transparent display and is disposed on the observation optical axis O2 of the observation system 13. The status indicator 60 indicates whether or not the light source 121 is turned on or off at a position that does not interfere with the observation of the subject eye E by the observation system 13. In FIG. 8, the status indicator 60 includes an ON/OFF indication point 61 near the upper end. The status indicator 60 does not interfere with the observation of the subject eye E by not displaying anything below the ON/OFF indication point 61.

The status indicator 60 indicates whether the light source 121 is turned on or off by letters, and makes it easy to grasp which state it is in. The ON/OFF indication point 61 according to the first embodiment is configured to display "LIGHT SOURCE OFF" when the light source 121 is turned off, and not display anything when the light source 121 is turned on. With this, the examiner can easily recognize whether the light source 121 is turned on or off by the ON/OFF indication point 61 while maintaining a condition of observing the subject eye E by the observation system 13. The status indicator 60 is not limited to the configuration of the first embodiment, but may be one with a different display mode or one with a display even when it is turned on or one with another configuration as long as the examiner as an observer can visually recognize whether the light source 121 is turned on or off.

(Operation of Light Intensity Adjustment) Next, operation to observe the subject eye E by using the slit-lamp microscope 10 will be described. The slit-lamp microscope 10 makes it possible to observe the subject eye E irradiated with the slit light with any brightness by suitably operating the observation magnification operation knob 13c, the brightness adjustment knob 14c and the slit opening/closing knob 125c.

At this time, in the slit-lamp microscope 10, for example, when the rotational operation has not been conducted on the slit opening/closing knob 125c or has been conducted on the slit opening/closing knob 125c in a range not to be the fully closed rotational position, the light intensity of the light source 121 is adjusted in accordance with the operation amount (rotational position) of the brightness adjustment knob 14c in Step S1 to proceed to Step S2. Then, in the slit-lamp microscope 10, when the slit opening/closing knob 125c is not at the fully closed rotational position, and then when the slit light that has been formed from the light source 121 via the pair of slit blades 125a, 125b is in a proper condition, the process proceeds in the order of Steps S3, S4 and S1. In this manner, the above operation is repeated, thereby making it possible to observe the subject eye E irradiated with the slit light with any brightness.

Here, in the slit-lamp microscope 10, when the slit opening/closing knob 125c has been rotated until the fully closed rotational position, the process proceeds in the order of Steps S1, S2, S3, and S5 and it determines whether or not it is set to emit an infrared light in the background illumination unit 20. In case that it is set to emit an infrared light in the background illumination unit 20, the process proceeds to Step S6. In Step S6, the light source 121 is turned off and the background illumination unit 20 is turned on. With this, in the slit-lamp microscope 10, when projecting infrared light, it is possible to prevent visible light from mixing therewith and it is possible to properly conduct observation and photographing of the meibomian gland by projecting infrared light. In the slit-lamp microscope 10, it is possible to turn into a condition of projecting infrared light by the background illumination unit 20 by only operating the slit opening/closing knob 125c. Therefore, it is possible to smoothly proceed to observation and photographing of the meibomian gland. In case that it is not set to emit infrared light in the background illumination unit 20, the process proceeds to Step S7 to turn off the light source 121. Therefore, in the slit-lamp microscope 10, in a condition that the pair of slit blades 125a, 125b is closed, it is possible to prevent seeing the light that escapes from a gap between the slit blades 125a, 125b, thereby giving an impression as having a high quality.

In the slit-lamp microscope 10, in the case that the rotational operation has been conducted on the slit opening/closing knob 125c in a range not to be the fully closed rotational position, but the generated slit light is not in a proper condition, the process proceeds in the order of Steps S 1, S2, S3, S4, and S5. In Step S5, it determines whether or not it is set to emit infrared light in the background illumination unit 20. In the slit-lamp microscope 10, in the case that it is set to emit infrared light in the background illumination unit 20, the process proceeds to Step S6. In Step S6, the light source 121 is turned off and the background illumination unit 20 is turned on. In contrast, in the case that it is not set to emit infrared light in the background illumination unit 20, the process proceeds to Step S7 to turn off the light source 121.

In this way, in the slit-lamp microscope 10, in the case that the generated slit light is not in a proper condition although the slit opening/closing knob 125c is not at the fully closed rotational position, it determines that the pair of slit blades 125a, 125b is substantially closed. As a result, the light source is turned off and the background illumination unit 20 is turned on. Here, in the slit-lamp microscope 10, in the case that the generated slit light is not in a proper condition, it determines that the slit opening/closing knob 125c is at a rotational position that is extremely close to the fully closed rotational position, since the two slit blades 125a, 125b are partially in contact with or extremely close to each other. Therefore, in this case, it is considered that the examiner has tried to substantially close the pair of slit blades 125a, 125b. Furthermore, in the slit-lamp microscope 10, it is difficult to conduct a proper observation of the subject eye E by projecting the slit light not in a proper condition. Thus, in the slit-lamp microscope 10, although the slit opening/closing knob 125c is not at the fully closed rotational position, it causes no problem to determine that the pair of slit blades 125a, 125b is substantially closed. As a result, in the slit-lamp microscope 10, it is possible to give an impression as the slit-lamp microscope 10 has high quality and to conduct an appropriate observation of the subject eye E using infrared light by turning off the light source 121 and turning on the background illumination unit 200 when the generated slit light is not in a proper condition.

Then, in the slit-lamp microscope 10, under a condition that the light source 121 is turned off and the background illumination unit 20 is suitably turned on, when the slit opening/closing knob 125c is rotated from the fully closed rotational position in a direction to open the slit, the process proceeds from Step S1 to Step S2 to turn on the light source 121 and suitably turn off the background illumination unit 20. Furthermore, in the slit-lamp microscope 10, under a condition that the light source 121 is turned off, when the slit opening/closing knob 125c is rotated from the fully closed rotational position in a direction to open the slit, the process proceeds from Step S1 to Step S2 to turn on the light source 121. Thus, in the slit-lamp microscope 10, irrespective of whether the background illumination unit 20 is turned on or not, it is possible to smoothly proceed to an observation of the subject eye E using the slit light by only operating the slit opening/closing knob 125c to open the pair of slit blades 125a, 125b from a condition that the light source 121 is turned off.

Next, the task of art of slit-lamp microscopes will be described. In conventional slit-lamp microscopes, it is possible to obtain a condition that the subject eye is not irradiated with the slit light by closing the pair of slit blades, that is, bringing the two slit blades into contact with each other to eliminate the slit, thereby blocking the light from the light source at the both slit blades. In such slit-lamp microscopes, however, unless extremely improving the processing and assembly precisions of the slit blades, even if the two slit blades are brought into contact with each other, it is difficult to achieve contact with a precision that can prevents the light from escaping in the entire area in the longitudinal direction of the slit. Therefore, in such slit-lamp microscopes, even if the two slit blades are moved to their contact position, a gap(s) is formed between the two slit blades. As a result, the light escaping from the gap(s) may reach the subject eye. With this, in such slit-lamp microscopes, the gap(s) formed between the two slit blades may be recognized by the examinee or examiner, thereby giving an impression as having a low quality.

In contrast, in the slit-lamp microscope 10 of the present invention, when the main controller 31 controlling the light source 121 determines that the pair of slit blades has been closed, the light source 121 is turned off. Therefore, in the slit-lamp microscope 10, when the two slit blades 125a, 125b have been moved to the position where the slit blades 125a, 125b are closed (where the slit blades 125a, 125b are in contact with each other), there is no light from the light source 121. Therefore, irrespective of the processing and assembly precisions of the slit blades 125a, 125b, it is possible to certainly prevent escaping of the light from the gap(s). With this, in the slit-lamp microscope 10, under the condition that the pair of slit blades 125a, 125b is closed, there is no light escaping from the gap(s) and reaching the subject eye E. This can prevent giving an impression as having a low quality. Furthermore, in the slit-lamp microscope 10, there is no need for processing and assembling the pair of slit blades 125a, 125b with an extremely high precision that enables preventing light escape in the entire area of the slit. Therefore, it is possible to prevent the increase of the cost and the production steps resulting from the increase of the processing and assembling precisions.

The slit-lamp microscope 10 according to the first embodiment of the present invention includes an illumination system 12 that is configured to project a slit light onto a subject eye E; a slit portion 125 that is configured to form the slit light having a width by passing a light from a light source 121 through a slit between a pair of the slit blades 125a, 125b in the illumination system 12; and a main controller 31 that is configured to control the light source 121. The main controller 31 is configured to turn the light source 121 off when the two slit blades 125a, 125b are closed. Therefore, when the two slit blades 125a, 125b are closed, there is no light from the light source 121. With this, irrespective of the processing and assembly precisions of the pair of slit blades 125a, 125b, it is possible to certainly prevent escaping of the light from the gap(s).

The slit-lamp microscope 10 further includes a slit distance detector 42 that is configured to detect a distance d between the two slit blades 125a, 125b. When the main controller 31 determines that the distance d between the two slit blades 125a, 125b is equal to or smaller than the set distance ds based on a detection result from the slit distance detector 42, the main controller 31 determines that the two slit blades 125a, 125b are closed to turn the light source 121 off. In this slit-lamp microscope 10, when the distance d is equal to or smaller than the set distance ds in a set positional relationship between the two slit blades 125a, 125b, it is determined that the two slit blades 125a, 125b are closed. Therefore, irrespective of the processing and assembly precisions of the pair of slit blades 125a, 125b, it is possible to turn the light source 121 off at a set position therebetween. This can prevent product-to-product variation.

The slit-lamp microscope 10 further includes a slit opening/closing knob 125c that is configured to adjust the distance d between the two slit blades 125a, 125b by a rotational operation thereof. The slit opening/closing knob 125c is configured such that a rotation resistance against the rotational operation of the slit opening/closing knob 125c partially changes when the slit opening/closing knob 125c is in a rotational position (the fully closed rotational position) to close the distance d between the two slit blades 125a, 125b. Therefore, in the slit-lamp microscope 10, the examiner operating the slit opening/closing knob 125c can recognize the fully closed rotational position by a finger sensation, thereby causing no sense of discomfort that the light source 121 was turned off. This may result from the examiner's imagination that, at the fully closed rotational position, the pair of the slit blades 125a, 125b should actually be fully closed, thereby causing the slit light disappear.

The slit-lamp microscope 10 further includes a slit light detector 45 that is configured to detect a condition of the slit light. The main controller 31 is configured to turn the light source 121 off when the main controller 31 determines that the condition of the slit light is out of a proper condition based on a detection result of the slit light detector 45. In the slit-lamp microscope 10, the light source 121 is turned off when it is determined that the condition of the slit light is out of a proper condition, in addition to when it is determined that the two slit blades 125a, 125b are closed. Therefore, in the slit-lamp microscope 10, when the two slit blades 125a, 125b are brought into a partial contact with each other or an extremely close position to each other, it is determined that the slit blades 125a, 125b are substantially closed and the light source 121 is turned off. This operation conforms to the examiner's intention and can prevent the examiner from not being able to properly observe the subject eye E.

The slit-lamp microscope 10 further includes an observation system 13 that is configured to observe or photograph a reflection light on the subject eye E; and a background illumination unit 20 that is configured to irradiate the subject eye E with an infrared light. In the slit-lamp microscope 10, the main controller 31 is configured to start irradiating the subject eye E with the infrared light by the background illumination unit 20 when the main controller 31 determines that the two slit blades 125a, 125b are closed to turn the light source 121 off. Therefore, it is possible to proceed to an infrared light irradiation condition by the background illumination unit 20 by only closing the two slit blades 125a, 125b. Furthermore, it is possible to conduct observation and the like by the infrared light irradiation while preventing visible light from mixing with the infrared light.

In the slit-lamp microscope 10, the main controller 31 is configured such that, when the two slit blades 125a, 125b are opened, the light source 121 is turned on and the infrared light irradiation by the background illumination unit 20 is stopped. Therefore, in the slit-lamp microscope 10, it is possible to smoothly proceed to an observation of the subject eye E by only opening the two slit blades 125a, 125b.

The slit-lamp microscope 10 further includes an observation system 13 that is configured to observe or photograph a reflection light reflected by the subject eye E. The observation system 13 includes a status indicator 60 that is configured to indicate a condition that the light source 121 is turned on or off. Therefore, in the slit-lamp microscope 10, while the examiner maintains a condition of observing the subject eye E by the observation system 13, the examiner can recognize the current lighting state of the light source 121, thereby alleviating discomfort caused by turning the light source 121 off.

As above, the slit-lamp microscope according to the first embodiment of the present invention has been described. However, the specific configuration of the present invention is not limited to the first embodiment. Changes and additions in design should be allowed as long as they do not deviate from the gist of the inventions recited in the claims.

In the first embodiment, the background illumination unit 20 capable of projecting infrared light is attached. However, it is not limited to the configuration of the first embodiment. The background illumination unit 20 may not be attached, or the background illumination unit 20 not projecting infrared light may be attached. In such a case, in the flowchart shown in FIG. 7, Steps S5 and S6 may be omitted and the process proceeds to Step S7 if YES in Step 3 and if NO in Step S4.

In the first embodiment, it is determined whether or not the acquired distance d (slit width) between the two slit blades 125a, 125b is equal to or smaller than the set distance ds by using the slit distance detector 42 that is configured to detect the distance d therebetween. However, it is not limited to the configuration of the first embodiment. It is optional to use a slit opening/closing detector that is configured to detect whether or not the two slit blades 125a, 125b are closed by another method.

### Reference Signs

10 SLIT-LAMP MICROSCOPE
12 ILLUMINATION SYSTEM
13 OBSERVATION SYSTEM
20 BACKGROUND ILLUMINATION UNIT
31 MAIN CONTROLLER (AN EXAMPLE OF CONTROLLER)
42 SLIT DISTANCE DETECTOR
45 SLIT LIGHT DETECTOR
60 STATUS INDICATOR
121 LIGHT SOURCE
125 SLIT PORTION
125a, 125b SLIT BLADES
125c SLIT OPENING/CLOSING KNOB
d DISTANCE
ds SET DISTANCE
E SUBJECT EYE

## Claims

1. A slit-lamp microscope (10) comprising:
an illumination system (12) that is configured to project a slit light onto a subject eye (E);
a slit portion (125) that is configured to generate the slit light having a width by passing a light from a light source (121) through a slit between a pair of slit blades (125a, 125b) in the illumination system (12); and
a controller (30, 31) that is configured to control the light source (121);
wherein the controller (30, 31) is configured to turn the light source (121) off when the pair of slit blades (125a, 125b) is closed.

2. The slit-lamp microscope (10) according to claim 1, further comprising:
a slit distance detector (42) that is configured to detect a distance (d) between the pair of slit blades (125a, 125b),
wherein when the controller (30, 31) determines that the distance (d) between the pair of slit blades (125a, 125b) is equal to or smaller than a set distance (ds) based on a detection result from the slit distance detector (42), the controller (30, 31) determines that the pair of slit blades (125a, 125b) is closed and turns off the light source (121).

3. The slit-lamp microscope (10) according to claim 2, further comprising:
a slit opening/closing knob (125c) that is configured to adjust the distance between the pair of slit blades (125a, 125b) by a rotational operation of the slit opening/closing knob (125c),
wherein the slit opening/closing knob (125c) is configured such that a rotation resistance against the rotational operation of the slit opening/closing knob (125c) partially changes when the slit opening/closing knob (125c) is in a rotational position at which the pair of slit blades (125a, 125b) are closed.

4. The slit-lamp microscope (10) according to claim 1, further comprising:
a slit light detector (45) that is configured to detect a condition of the slit light,
wherein the controller (30, 31) is configured to turn off the light source (121) when the controller (30, 31) determines that the condition of the slit light is not in a proper condition based on a detection result from the slit light detector (45).

5. The slit-lamp microscope (10) according to claim 1, further comprising:
a background illumination unit (20) that is configured to irradiate the subject eye (E) with an infrared light,
wherein the controller (30, 31) is configured to start irradiating the subject eye (E) with the infrared light by the background illumination unit (20) when the pair of slit blades (125a, 125b) are closed and the light source (121) is turned off.

6. The slit-lamp microscope (10) according to claim 5, wherein the controller (30, 31) is configured such that, when the pair of slit blades (125a, 125b) are opened, the light source (121) is turned on and the infrared light irradiation by the background illumination unit (20) is stopped.

7. The slit-lamp microscope (10) according to any one of claims 1 to 6, further comprising:
an observation system (13) that is configured to observe or photograph a reflection light reflected by the subject eye (E),
wherein the observation system (13) comprises a status indicator (60) that is configured to indicate a condition that the light source (121) is turned on or off.
